# EUROPEAN PATENT SPECIFICATION

(11) **EP 3 024 929 B1**
(45) Date of publication and mention of the grant of the patent: **16.10.2024**
(21) Application number: 14741337.1
(22) Date of filing: 21.07.2014
(51) Int. Cl.: C12N 9/02, C12N 15/53, C12N 15/82, A01H 5/02, A01H 5/10

(54) **DOWNY MILDEW RESISTANCE PROVIDING GENES IN SUNFLOWER**
GENE ZUR HERSTELLUNG EINER BESTÄNDIGKEIT GEGEN FLAUMIGEN MEHLTAU BEI SONNENBLUMEN
GÈNES DE RÉSISTANCE AU MILDIOU CHEZ LE TOURNESOL

(30) Priority: 22.07.2013 WO PCT/EP2013/065397
(43) Date of publication of application: 01.06.2016
(73) Proprietor: Enza Zaden Beheer B.V., 1602 DB Enkhuizen (NL)
(72) Inventor: VAN SCHIE, Christianus Cornelis Nicolaas, 1025 WH Amsterdam (NL); ZEILMAKER, Tieme, 3823 JJ Amersfoort (NL)
(74) Representative: van Kooij, Adriaan
(86) International application number: PCT/EP2014/065641
(87) International publication number: WO 2015/011101

(56) References cited:
- WO-A1-2008/092505
- SABETTA WILMA ET AL: "sunTILL: a TILLING resource for gene function analysis in sunflower", PLANT METHODS, vol. 7, June 2011 (2011-06-01), XP002730704, ISSN: 1746-4811
- RADWAN OSMAN ET AL: "Molecular Characterization of Two Types of Resistance in Sunflower to Plasmopara halstedii, the Causal Agent of Downy Mildew", PHYTOPATHOLOGY, vol. 101, no. 8, August 2011 (2011-08-01), pages 970 - 979, XP002730705, ISSN: 0031-949X
- FRANCHEL JEROME ET AL: "Positional cloning of a candidate gene for resistance to the sunflower downy mildew, Plasmopara halstedii race 300", THEORETICAL AND APPLIED GENETICS, vol. 126, no. 2, February 2013 (2013-02-01), pages 359 - 367, XP002730706, ISSN: 0040-5752
- VAN DAMME MIREILLE ET AL: "Arabidopsis DMR6 encodes a putative 2OG-Fe(II) oxygenase that is defense-associated but required for susceptibility to downy mildew.", THE PLANT JOURNAL : FOR CELL AND MOLECULAR BIOLOGY JUN 2008, vol. 54, no. 5, June 2008 (2008-06-01), pages 785 - 793, XP002730707, ISSN: 1365-313X

## Description

The present invention relates to downy mildew resistant genes in sunflower and especially to downy mildew resistance sunflower plants. The present invention further relates to methods for obtaining the present downy mildew resistant sunflower plants and the use of the present genes for providing downy mildew resistance in sunflower.

*Helianthus L.* is a genus of plants comprising about 52 species in the *Asteraceae* family. The common designation "sunflower" is generally used to indicate the annual species *Helianthus annuus. Helianthus annuus* and other species such as Jerusalem artichoke (*Helianthus tuberosus*), are cultivated in temperate regions as food crops and ornamental plants. The domesticated sunflower, *Helianthus annuus,* is the most familiar species of the *Helianthus L.* genus. *Helianthus annuus* is cultivated both for ornamental purposes as for providing vegetable oil from seeds.

Downy mildew, a common and destructive disease in sunflowers, is capable of killing or stunting plants, reducing stand and causing significant yield loss (up to 50 to 95%). Sunflowers are most susceptible to downy mildew in fields where heavy rain has fallen within 2-3 weeks after planting.

Downy mildew refers to any of several types of oomycete plant pathogens that are obligate parasites of plants. Downy mildews exclusively belong to *Peronosporaceae.* The downy mildew pathogen generally causing downy mildew disease in cultivated sunflowers is designated Plasmopara *halstedii* or *Plasmopara helianthi.*

In the technical field of sunflower cultivation and breeding, there is a constant need to identify new resistance genes against downy mildew. However, most resistance gene identified are monogenic dominant resistance genes and the resistance provided by these genes is generally rapidly broken because downy mildew pathogens evolve and adapt at a high frequency thereby regaining the ability to successfully infect a host plant. Accordingly, there is a continuous need in de art for new resistance genes, preferably resistance genes of which the resistance is not readily broken by adaptation of the pathogen.

A disadvantage of known sunflower resistance genes is that, besides providing pathogen resistance, these genes often are accompanied by undesired phenotypes such as stunted growth or spontaneous occurrence cell death. Accordingly, there is a continuous need in the art for new resistance genes not providing, besides the resistance, undesirable phenotypes.

It is an object of the present invention, amongst other objects, to meet, at least partially if not fully, the above needs of the art.

This object of the present invention, amongst other objects, is met by providing a sunflower plants and resistance genes as outlined in the appended claims.

Specifically, this object of the present invention, amongst other objects, is met, according to a first aspect, by sunflower plants being resistant to the plant pathogen downy mildew, wherein the present plant comprises a downy mildew resistance conferring gene encoding (a) protein(s) comprising the amino acid sequence as shown in SEQ ID No. 2 and/or SEQ ID NO. 4 or a downy mildew resistance conferring gene encoding a protein with more than 90% sequence identity, preferably more than 94% sequence identity, more preferably more than 96% sequence identity with identity SEQ ID No. 2 and/or SEQ ID NO. 4 and wherein the expression of the present resistance conferring gene is reduced as compared to the expression of the present resistance conferring gene in a sunflower plant not being resistant to the plant pathogen downy mildew or the enzymatic activity of the present protein is reduced as compared to the enzymatic activity of the present protein in a sunflower plant not being resistant to the plant pathogen downy mildew.

In the research that led to the present invention, it was surprisingly found that a reduced expression of the present genes or a reduced enzymatic activity of the present proteins provided a broad and durable resistance to downy mildew in sunflower plants.

According to the present invention, an expression is reduced in comparison with the expression of the present resistance conferring gene in a sunflower plant not being resistant to the plant pathogen downy mildew. The term "not being resistant" indicates a resistance level, determined in an appropriate disease test and using an appropriate reference plant such as a parent plant, being less than the resistance level observed in the present plants. Accordingly, the present resistance can also be designated as an increased resistance to downy mildew. Suitable reference plants according to the invention, besides parent plants, can also be plants generally designated in the art as downy mildew susceptible plants.

A suitable disease test is inoculating plants with a downy mildew pathogen and subsequently observing the occurrence of disease symptoms such as large, angular or blocky, yellow areas visible on the upper surface of leaves or destroyed leaf tissue.

Expression levels in the present plants and the reference plants can be determined using any suitable and generally known Molecular Biology technique such as a quantitative Polymerase Chain Reaction (PCR) or mRNA hybridization.

According to the present invention, an enzymatic activity is reduced in comparison with the activity of the present protein in a sunflower plant not being resistant to the plant pathogen downy mildew. The term "not being resistant" indicates a resistance level, determined in an appropriate disease test and using an appropriate reference plant, such as a parent plant, being less than the resistance level observed in the present plants. Accordingly, the present resistance can also be designated as an increased resistance to downy mildew. Suitable reference plants according to the invention can, besides parent plants, also be plants generally designated in the art as downy mildew susceptible plants. A suitable disease test is inoculating plants with downy mildew and subsequently observing the occurrence of disease symptoms such as large, angular or blocky, yellow areas visible on the upper surface of leaves or destroyed leaf tissue.

The present proteins have a 2-oxoglutarate FE(II)-dependent oxygenase activity. The enzyme has an absolute requirement for Fe(II) and catalyzes two-electron oxidations, including hydroxylation, desaturation and oxidative ring closure reactions. The oxidation of the 'prime' substrate is coupled to the conversion of 2OG into succinate and CO₂. One of the oxygens of the dioxygen molecule is incorporated into succinate. In the case of desaturation reactions, the other dioxygen-derived oxygen is presumably converted to water. In hydroxylation reactions, the partial incorporation of oxygen from dioxygen into the alcohol product occurs with significant levels of exchange of oxygen from water being observed. Accordingly, the present reduced activity can be determined using an assay measuring compounds being either the starting compounds or the resulting compounds of the enzymatic reaction. As a suitable alternative, protein levels, being inherently indicative of a reduced activity, of the present proteins can be determined by, for example, ELISA or protein hybridization both being techniques commonly known to the skilled person.

Within the context of the present invention, resistance to downy mildew is individually or in combination provided, through reduced expression or activity, to the present sunflower plants by the present proteins or genes encoding the present proteins.

The present sunflower plants can be obtained by mutagenesis of downy mildew susceptible plant or downy mildew resistant plants thereby increasing the resistance thereof. For example, mutations, either at the expression level or the protein level, can be introduced in these plants by using mutagenic chemicals such as ethyl methane sul2fonate (EMS) or by irradiation of plant material with gamma rays or fast neutrons. The resulting mutations can be directed or random. In the latter case, mutagenized plants carrying mutations in the present resistance conferring genes can be readily identified by using the TILLING (Targeting Induced Local Lesions IN Genomes) method (McCallum et al. (2000) Targeted screening for induced mutations. Nat. Biotechnol. 18, 455-457, and Henikoff et al. (2004) TILLING. Traditional mutagenesis meets functional genomics. Plant Physiol. 135, 630-636). Briefly, this method is based on the PCR amplification of a gene of interest from genomic DNA of a large collection of mutagenized plants in the M2 generation. By DNA sequencing or by scanning for point mutations using a single-strand specific nuclease, such as the CEL-I nuclease (Till et al. (2004) Mismatch cleavage by single-strand specific nucleases. Nucleic Acids Res. 32, 2632-2641) individual plants having a mutation in the present genes are identified.

According to a preferred embodiment of this first aspect of the present invention, the present downy mildew pathogens are *Plasmopara halstedii* and/or *Plasmopara helianthi.* However, other pathogens belonging to the *Peronosporaceae* and capable of causing downy mildew disease in sunflower are contemplated within the context of the present invention.

According to another preferred embodiment of this first aspect of the present invention, the present reduced enzymatic activity is provided by one or more mutations in the coding sequence of the present genes resulting in a truncated or non-functional protein. Truncated proteins can be readily determined by analyzing gene transcripts at the mRNA or cDNA level and non-functional proteins can be determined in enzyme assays or using conformation-dependent antibodies. Mutations which can be assayed at the transcript level are, for example, amino acid substitutions, frame-shifts or pre-mature stop codons.

According to an especially preferred embodiment of this first aspect of the present invention, the present mutations resulting in a reduced activity of the present proteins are mutations resulting in the absence of or amino acid substitution(s) in the sequence motif "WRDYLR" or Trp-Arg-Asp-Tyr-Leu-Arg of the coding sequence of the present resistance providing gene. The present sequence motif can be found at amino acid positions 107 to 112 of SEQ ID No. 2 and at amino acid positions 116 to 121 of SEQ ID No. 4. The present inventors have found that mutations in this region especially affect the downy mildew resistance phenotype, i.e. level of resistance, observed. Especially mutations involving Y (Tyr) and/or R (Arg) are highly correlated the downy mildew resistance phenotype, i.e. level of resistance, observed.

According to yet another preferred embodiment this first aspect of the present invention, the present reduced expression is provided by one or more mutations in the regulatory regions or non-coding sequences of the present genes. Examples of regulatory regions of the present genes are promotor and terminator regions and examples of non-coding regions are introns and especially splicing influencing motifs therein.

According to a second aspect, the present invention provides seeds, plant tissues or plants parts of the sunflower plants as described above or obtainable from the sunflower plants as described above, comprising a downy mildew resistance conferring gene encoding a protein comprising the amino acid sequence as shown in SEQ ID No. 2 and/or SEQ ID NO. 4 or a downy mildew resistance conferring gene encoding a protein with more than 90% sequence identity, preferably more than 94% sequence identity, more preferably more than 96% sequence identity with identity SEQ ID No. 2 and/or SEQ ID NO. 4 and the expression of the resistance conferring gene is reduced as compared to the expression of the resistance conferring gene in a sunflower plant not being resistant to the plant pathogen downy mildew or the enzymatic activity of the protein is reduced as compared to the enzymatic activity of the protein in a sunflower plant not being resistant to the plant pathogen downy mildew.

According to a third aspect, the present invention relates to methods for providing sunflower plants being resistant, or methods for increasing the resistance of sunflower plants, to the plant pathogen downy mildew wherein the present methods comprise the step of introducing in a sunflower plant a downy mildew resistance conferring gene encoding a protein comprising the amino acid sequence as shown in SEQ ID No. 2 and/or SEQ ID NO. 4 or a downy mildew resistance conferring gene encoding a protein with more than 90% sequence identity, preferably more than 94% sequence identity, more preferably more than 96% sequence identity with identity SEQ ID No. 2 and/or SEQ ID NO. 4 and the expression of the resistance conferring gene is reduced as compared to the expression of the resistance conferring gene in the starting sunflower plant or the enzymatic activity of the protein is reduced as compared to the enzymatic activity of the protein in the starting sunflower plant not being resistant to the plant pathogen downy mildew.

According to a fourth aspect, the present invention relates to the use of a gene, or the cDNA sequence thereof, encoding a protein comprising the amino acid sequence as shown in SEQ ID No. 2 or SEQ ID NO. 4 or a downy mildew resistance conferring gene encoding a protein with more than 90% sequence identity, preferably more than 94% sequence identity, more preferably more than 96% sequence identity with identity SEQ ID No. 2 or SEQ ID NO. 4 for providing sunflower plants being resistant or having an increased resistance to the plant pathogen downy mildew.

According to a fifth aspect, the present invention relates to proteins having an amino acid sequence comprising SEQ ID No. 2 or SEQ ID No. 4.

According to a sixth aspect, the present invention relates to nucleic acid sequences comprising SEQ ID No. 1 or SEQ ID N0. 3.

According to a seventh aspect, the present invention relates to gene encoding a protein having an amino acid sequence comprising SEQ ID No. 2 or SEQ ID No. 4 or a nucleic acid sequence comprising SEQ ID No. 1 or SEQ ID N0. 3.

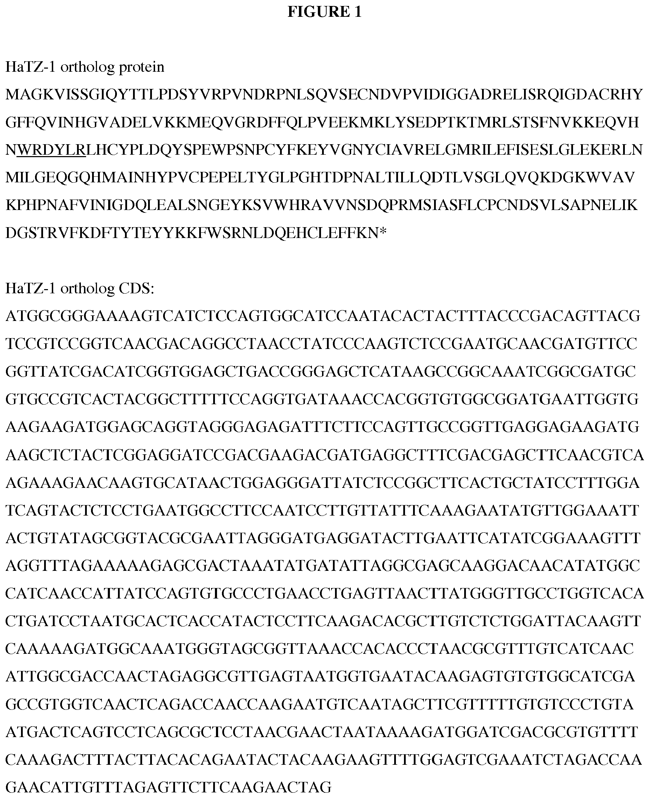

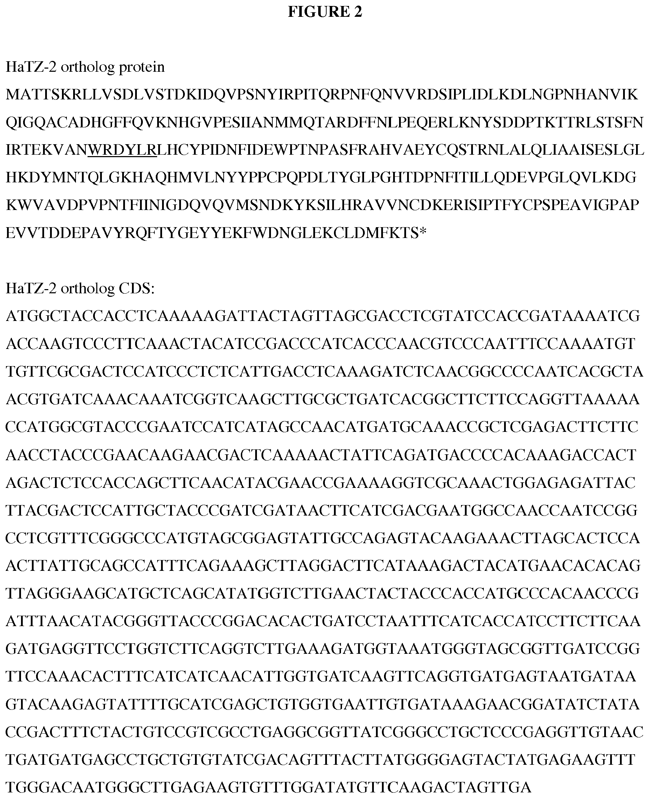

## Claims

1. Sunflower plant being resistant to the plant pathogen downy mildew, wherein said plant comprises a downy mildew resistance conferring gene encoding a protein comprising the amino acid sequence as shown in SEQ ID No. 2 or SEQ ID No. 4 or a downy mildew resistance conferring gene encoding a protein with more than 90% sequence identity, preferably more than 94% sequence identity, more preferably more than 96% sequence identity with identity SEQ ID No. 2 or SEQ ID No. 4 and wherein the enzymatic activity of said protein is reduced as compared to the enzymatic activity of said protein in a sunflower plant not being resistant to the plant pathogen downy mildew, wherein said reduced enzymatic activity is provided by one or more amino acid substitutions in the sequence motif "WRDYLR" of the coding sequence of said resistance providing gene.

2. Sunflower plant according to claim 1, wherein the downy mildew plant pathogen is *Plasmopara halstedii* or *Plasmopara helianthi.*

3. Seeds, plant tissue or plants parts of a sunflower plant as defined in claim 1 or claim 2 comprising a downy mildew resistance conferring gene encoding a protein comprising the amino acid sequence as shown in SEQ ID No. 2 or SEQ ID No. 4 or a downy mildew resistance conferring gene encoding a protein with more than 90% sequence identity, preferably more than 94% sequence identity, more preferably more than 96% sequence identity with identity SEQ ID No. 2 or SEQ ID No. 4 and wherein the enzymatic activity of said protein is reduced as compared to the enzymatic activity of said protein in a sunflower plant not being resistant to the plant pathogen downy mildew, wherein said reduced enzymatic activity is provided by one or more amino acid substitutions in the sequence motif "WRDYLR" of the coding sequence of said resistance providing gene.

## Patentansprüche

1. Sonnenblumenpflanze, die gegen das Pflanzenpathogen Falscher Mehltau resistent ist, wobei die Pflanze ein Falscher-Mehltau-Resistenz verleihendes Gen umfasst, das ein Protein codiert, umfassend die Aminosäuresequenz, wie in SEQ ID Nr. 2 oder SEQ ID Nr. 4 gezeigt, oder ein Falscher-Mehltau-Resistenz verleihendes Gen, das ein Protein mit mehr als 90 % Sequenzidentität, vorzugsweise mehr als 94 % Sequenzidentität, mehr bevorzugt als 96 % Sequenzidentität mit der Identität SEQ ID Nr. 2 oder SEQ ID Nr. 4 codiert, und wobei die enzymatische Aktivität des Proteins im Vergleich zu der enzymatischen Aktivität des Proteins in einer Sonnenblumenpflanze reduziert ist, die nicht gegen das Pflanzenpathogen Falscher Mehltau resistent ist, wobei die reduzierte enzymatische Aktivität durch eine oder mehrere Aminosäuresubstitutionen in dem Sequenzmotiv "WRDYLR" der codierenden Sequenz des Resistenz bereitstellenden Gens bereitgestellt wird.

2. Sonnenblumenpflanze nach Anspruch 1, wobei das Falscher-Mehltau-Pflanzenpathogen *Plasmopara halstedii* oder *Plasmopara helianthi* ist.

3. Samen, Pflanzengewebe oder Pflanzenteile einer Sonnenblumenpflanze nach Anspruch 1 oder 2, umfassend ein Falscher-Mehltau-Resistenz verleihendes Gen, das ein Protein codiert, umfassend die Aminosäuresequenz, wie in SEQ ID Nr. 2 oder SEQ ID Nr. 4 gezeigt, oder ein Falscher-Mehltau-Resistenz verleihendes Gen, das ein Protein mit mehr als 90 % Sequenzidentität, vorzugsweise mehr als 94 % Sequenzidentität, mehr bevorzugt als 96 % Sequenzidentität mit der Identität SEQ ID Nr. 2 oder SEQ ID Nr. 4 codiert, und wobei die enzymatische Aktivität des Proteins im Vergleich zu der enzymatischen Aktivität des Proteins in einer Sonnenblumenpflanze reduziert ist, die nicht gegen das Pflanzenpathogen Falscher Mehltau resistent ist, wobei die reduzierte enzymatische Aktivität durch eine oder mehrere Aminosäuresubstitutionen in dem Sequenzmotiv "WRDYLR" der codierenden Sequenz des Resistenz bereitstellenden Gens bereitgestellt wird.

## Revendications

1. Plante de tournesol résistante à l'agent pathogène du mildiou, dans laquelle ladite plante comprend un gène conférant une résistance au mildiou codant une protéine comprenant la séquence d'acides aminés telle qu'indiquée dans SEQ ID No. 2 ou SEQ ID No. 4 ou un gène conférant une résistance au mildiou codant une protéine ayant plus de 90 % d'identité de séquence, de préférence plus de 94 % d'identité de séquence, plus préférablement plus de 96 % d'identité de séquence avec une identité SEQ ID No. 2 ou SEQ ID No. 4 et dans laquelle l'activité enzymatique de ladite protéine est réduite par rapport à l'activité enzymatique de ladite protéine dans une plante de tournesol non résistante à l'agent pathogène du mildiou, dans laquelle ladite activité enzymatique réduite est fournie par une ou plusieurs substitutions d'acides aminés dans le motif de séquence « WRDYLR » de la séquence codante dudit gène conférant la résistance.

2. Plante de tournesol selon la revendication 1, dans laquelle l'agent pathogène du mildiou est *Plasmopara halstedii* ou *Plasmopara helianthi.*

3. Graines, tissus végétaux ou parties de plantes d'une plante de tournesol telle que définie dans la revendication 1 ou la revendication 2, comprenant un gène conférant une résistance au mildiou codant une protéine comprenant la séquence d'acides aminés telle qu'indiquée dans SEQ ID No. 2 ou SEQ ID No. 4 ou un gène conférant une résistance au mildiou codant une protéine ayant plus de 90 % d'identité de séquence, de préférence plus de 94 % d'identité de séquence, plus préférablement plus de 96 % d'identité de séquence avec une identité SEQ ID No. 2 ou SEQ ID No. 4 et dans lesquels l'activité enzymatique de ladite protéine est réduite par rapport à l'activité enzymatique de ladite protéine dans une plante de tournesol non résistante à l'agent pathogène du mildiou, dans lesquels ladite activité enzymatique réduite est fournie par une ou plusieurs substitutions d'acides aminés dans le motif de séquence « WRDYLR » de la séquence codante dudit gène conférant la résistance.
